# EUROPÄISCHE PATENTANMELDUNG

(11) **EP 1 223 538 A2**
(43) Veröffentlichungstag der Anmeldung: **17.07.2002**
(21) Anmeldenummer: 01128858.6
(22) Anmeldetag: 04.12.2001
(51) Int. Cl.: G06K 9/00, G07C 9/00, G06F 3/00

(54) **Handerkennung mit Positionsbestimmung**

(30) Priorität: 09.01.2001 DE 10100615
(71) Anmelder: SIEMENS AKTIENGESELLSCHAFT, 80333 München (DE)
(72) Erfinder: Heger, Hans Jörg, 80807 München (DE); Küpper, Wolfgang, Dr., 80796 München (DE)

(57) **Zusammenfassung**

Die Erfindung betrifft eine Anordnung, ein Verfahren und ein Programmprodukt zur Erfassung einer an einem Arm befindlichen Hand, bei der zumindest teilweise ein Abbild der Hand und des Arms aufnehmbar ist. Die Anordnung weist Mittel zum Auffinden eines Teils des Abbilds mit einem großen Verhältnis von Inhalt zu Außenseiten auf.

## Beschreibung

Die Erfindung betrifft eine Anordnung, ein Verfahren und ein Programmprodukt zur Erfassung einer an einem Arm befindlichen Hand, wobei zumindest teilweise ein Abbild der Hand und des Arms aufnehmbar ist.

Solche Anordnungen sind zum Beispiel aus US 5 533 177, US 5 751 843, US 5 828 779, EP 0 560 779 B1, EP 0 713 592 B1, EP 0 800 145 A2 und WO 98/38533 bekannt. Dabei wird ein Abbild der Hand und des Arms in Form eines digitalen Bildes aufgenommen.

Zumindest vom Arm wird das Abbild in der Regel teilweise aufgenommen, so dass nicht der ganze Arm abgebildet wird.

Während die oben genannten Anordnungen der Steuerung einer Datenverarbeitungsanlage über eine Benutzerschnittstelle dienen, existieren auch Anordnungen, die sich zur Authentisierung von Benutzern biometrischer Verfahren bedienen, das heißt, der Benutzer wird anhand von Körpermerkmalen oder charakteristischen Verhaltensweisen erkannt. Ein etabliertes biometrisches Verfahren ist die Authentisierung durch die Erkennung der Form der Hand oder eines Teiles der Hand.

Dabei wird die Hand des Benutzers in Anordnungen nach dem Stand der Technik zur Handerkennung auf eine vorgefertigte Platte mit Positionierungshilfen aufgelegt. Die Position der Hand wird durch die physikalischen Hilfsmittel, die beispielsweise als kleine Stahlbolzen ausgebildet sind, bis ins Detail festgelegt. Anschließend wird ein Abbild der statisch liegenden Hand erfasst und weiterverarbeitet. Auf der Grundlage dieses Abbildes wird die Authentisierung des Benutzers durchgeführt.

Es sind neben diesen Anordnungen und ihren Abwandlungen zwei weitere, in wesentlichen Punkten unterschiedliche Verfahren veröffentlicht. Beide Verfahren kommen ohne die Einschränkung der Festlegung der genauen Handposition aus. In EP 0 150 697 A1 wird eine berührungslose Messung erreicht, indem die Hand in annähernd parallele Lichtstrahlen gehalten wird. Hierfür weist die Anordnung eine spezielle Kammer auf, in welche die Hand gehalten wird.

In US 4 720 869 darf die Hand ohne physikalische Einschränkungen auf eine Glasplatte gelegt werden. Allerdings wird durch das zur Aufnahme des Abbildes der Hand verwendete Verfahren die Richtung implizit vorgegeben. Die Hand kann also auch hier nicht völlig frei aufgelegt werden.

Allen Anordnungen ist gemeinsam, dass sie über ihre Größe und ihren Aufbau sicherstellen, dass nur die Handkontur registriert wird. Die Anordnungen sind für handgroße Objekte dimensioniert und es wird die vom Gerät gelieferte Kontur vollständig als Handkontur interpretiert.

Davon ausgehend liegt der Erfindung die Aufgabe zugrunde, die einschränkende Festlegung der Handposition bei der Handerkennung aufzuheben und damit benutzerfreundliche Anordnungen und Verfahren zur Verfügung zu stellen.

Diese Aufgabe wird durch eine Anordnung, ein Verfahren und ein Programmprodukt mit den Merkmalen der unabhängigen Ansprüche gelöst.

Demnach weist die Anordnung Mittel zum Auffinden eines Teils des Abbilds auf, in das ein Objekt mit einem großen Verhältnis von Inhalt zu Außenseiten einbeschreibbar ist. Je nachdem, ob es sich um ein zwei- oder dreidimensionales Abbild handelt, ist mit dem Begriff Inhalt die Fläche oder das Volumen und mit dem Begriff Außenseiten der Umfang oder die Oberfläche des Teils gemeint.

Der Erfindung liegt die Erkenntnis zugrunde, dass die Hand, genauer genommen der Handteller, den kompaktesten Bereich in einer Kombination aus Arm, Hand und Fingern darstellt. Im Vergleich dazu sind Arm und Finger eher langgestreckt. Das Verhältnis der Größe ihres Inhalts zu ihren Außenseiten ist dementsprechend gering. Bei einem dreidimensionalen Abbild heißt das, dass das Verhältnis von Volumen zu Oberfläche bei Arm und Fingern sehr viel geringer ist als bei der Hand. Im zweidimensionalen Fall ist das Verhältnis von Fläche zu Umfang bei Arm und Fingern sehr viel geringer als bei der Hand.

Wie groß das Verhältnis für eine sichere Erfassung der Hand sein muss, wird durch die Anatomie der zu authentisierenden Personen bestimmt. Hier sind für unterschiedliche Kulturkreise und unterschiedliche Altersgruppen eventuell differierende Werte zu wählen. Im Allgemeinen kann eine sichere Erfassung durch einen passend gewählten Schwellwert für das Verhältnis gewährleistet werden.

Alternativ kann auf einen solchen Schwellwert aber auch verzichtet werden, wenn das große Verhältnis von Inhalt zu Außenseiten in etwa das größte Verhältnis von Inhalt zu Außenseiten ist, das im Abbild auftaucht. Diesem Vorgehen liegt die Annahme zugrunde, dass die Hand den absolut gesehen kompaktesten Teil in der Kombination Arm, Hand und Finger bildet.

Ein Auffinden des Teils des Abbilds mit einem großen Verhältnis von Inhalt zur Außenseiten ist ausgehend von dem Abbild schwierig in einem Algorithmus zur Datenverarbeitung implementierbar. Dazu müssen in dem Abbild zunächst in sinnvoller Weise unterschiedliche Teile identifiziert werden und danach jeweils das Verhältnis bestimmt werden. Eine derart ausgebildete Anordnung kann mit Hilfe eines neuronalen Netzes realisiert werden.

Bevorzugt wird aufgrund der einfacheren und weniger Rechenleistung benötigenden Realisierung aber die umgekehrte Vorgehensweise, bei der nicht vom Abbild ausgegangen wird, sondern ein geometrisches Objekt mehrfach dem Abbild einbeschrieben wird. Üblicherweise wird das geometrische Bild dem Abbild mehrfach einbeschrieben oder, was nur eine andere Formulierung desselben Sachverhalts ist, es werden dem Abbild mehrere geometrische Objekte einbeschrieben. Der Teil des Abbilds wird dann dort aufgefunden, wo das geometrische Objekt am größten einbeschreibbar ist oder, in der anderen Formulierung, wo das größte der geometrischen Objekte einbeschrieben werden kann. Bei diesem Füllalgorithmus bleibt das geometrische Objekt in seiner Grundform vorzugsweise identisch und wird nur in seinen Abmessungen verändert.

In der euklidischen Metrik weisen im zweidimensionalen Fall der Kreis und im dreidimensionalen Fall die Kugel das größte Verhältnis von Inhalt zur Außenseiten auf. Dementsprechend ist das geometrische Objekt im Fall eines zweidimensionalen Abbilds vorzugsweise ein Kreis. Dies hat sich insbesondere bewährt, wenn das Abbild in Bezug zur Hand von oben oder von unten aufgenommen wird. Alternativ zum Kreis ist aber auch ein Rechteck oder geeignet gewähltes Polygon verwendbar.

Im dreidimensionalen Fall kann entsprechend eine Kugel verwendet werden. Hier sind aber auch der Hand angepasstere Füllkörper als geometrische Objekte verwendbar, wie zum Beispiel ein Rotationskörper in Form einer konvexen Linse. Alternativ lässt sich hier aber auch ein Quader verwenden.

Vorzugsweise weist die Anordnung Mittel zum Bestimmen der Position der Hand aufgrund der Position des Teils des Abbilds auf. Hierbei kann die Handposition absolut im von der Anordnung aufgenommenen Aufnahmeraum bestimmt werden und/oder relativ gegenüber dem Arm und/oder den Fingern bzw. Teilen davon.

Letzteres ist für die Handerkennung, also für die Authentisierung der zur Hand gehörigen Person, vorteilhaft, weil sich so charakteristische Merkmale der Hand identifizieren lassen.

Weiterhin kann das Handzentrum bestimmbar sein, etwa indem es mit dem Mittelpunkt des einbeschriebenen geometrischen Objektes identifiziert wird. Nach der Erfassung des Handzentrums kann die Kontur auf weitere charakteristische Handmerkmale in der Umgebung des Zentrums untersucht werden.

Sollten weitere Körperteile oder andere Gegenstände von der Anordnung aufgenommen worden sein, so ist es vorteilhaft, wenn diese Mittel zur Identifizierung des Abbilds von Hand und Arm und insbesondere der Hand in einer Umgebung aufweist. So lässt sich beispielsweise ein ausgestreckter Arm leicht anhand seiner Proportionen von Länge zu Breite erkennen.

Bei einem Verfahren zum Erfassen einer an einem Arm befindlichen Hand wird zumindest teilweise ein Abbild der Hand und des Arms aufgenommen und dann ein Teil des Abbilds mit einem großen Verhältnis von Inhalt zu Außenseiten aufgefunden. Das Verfahren lässt sich sinngemäß durch die zur Anordnung in der Beschreibung und den Ansprüchen genannten Merkmale vorteilhaft ausgestalten.

Ein Programmprodukt für eine Datenverarbeitungsanlage, das Softwarecodeabschnitte enthält, mit denen ein solches Verfahren auf der Datenverarbeitungsanlage ausgeführt werden kann, lässt sich durch geeignete Implementierung des Verfahrens in einer Programmiersprache ausführen. Die Softwarecodeabschnitte werden dazu gespeichert. Dabei wird unter einem Programmprodukt das Programm als handelbares Produkt verstanden. Es kann in beliebiger Form vorliegen, so zum Beispiel auf Papier, einem computerlesbaren Datenträger oder über ein Netz verteilt.

Die Anordnung lässt sich zum Beispiel durch entsprechendes Programmieren und Einrichten einer Datenverarbeitungsanlage realisieren.

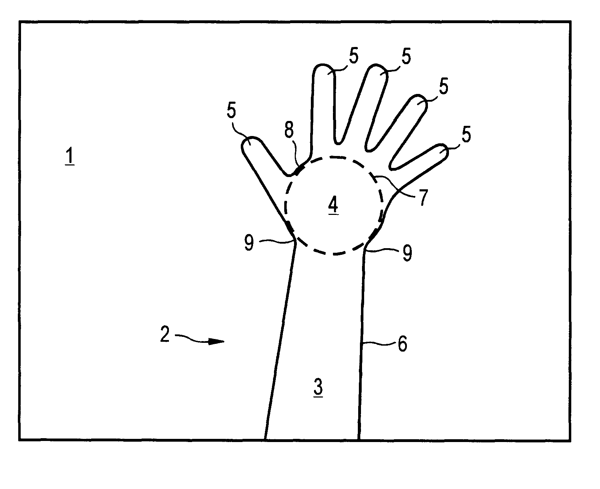

Weitere wesentliche Merkmale und Vorteile der Erfindung ergeben sich aus der Beschreibung eines Ausführungsbeispiels anhand der Zeichnung. Dabei zeigt
- Figur 1: ein Abbild von Hand und Arm in einem aufgenommenen Bereich.

In einer hier beispielhaft dargestellten Ausführungsform enthält die Anordnung einen Aufnahmeraum in Form einer Aufnahmefläche von etwa 100 x 80 cm, die von einer Videokamera aufgenommen wird. Zur Vermeidung von Umgebungseffekten, wie Sonneneinstrahlung oder Schattenbildung, wird die Szene ca. auf der Kameraachse mit Infrarotlicht beleuchtet und die Videokamera arbeitet mit einem Infrarotfilter. Eine weitere Erhöhung der Aufnahmegenauigkeit wird durch einen retroreflektierenden Untergrund erzielt. Aufgrund der Größe der Aufnahmefläche werden die Hand, die Finger und ein wechselnd großer Anteil des Armes eines Benutzers erfasst.

In Figur 1 ist das von der Videokamera aufgenommene Bild dargestellt. Dieses Bild wird in digitalisierter Form zur Verarbeitung an eine Datenverarbeitungsanlage weitergeleitet. Es enthält die Aufnahmefläche 1 sowie ein teilweises Abbild 2 von Arm 3 und Hand 4.

Im dargestellten Ausführungsbeispiel ist dieses Abbild zweidimensional. Es sind aber auch dreidimensionale Bilder denkbar, indem mit mehreren Videokameras oder anderen Aufnahmegeräten gearbeitet wird und aus den davon erhaltenen Daten ein dreidimensionales Abbild erzeugt wird.

Alternativ können zum Beispiel auch mit zwei Videokameras zwei zweidimensionale Abbilder erzeugt werden, um die Authentisierungsgenauigkeit zu erhöhen.

Für die Handerkennung ist im Wesentlichen nur die Kontur 6 von Arm 3, Hand 4 und Fingern 5 entscheidend, so dass das Abbild 2 nur diese Kontur 6 enthält.

Das Abbild 2 wird an eine Datenverarbeitungsanlage weitergeleitet, die so eingerichtet ist, dass ein Verfahren mit den folgenden Schritten ausgeführt wird.

In einem ersten Schritt wird das Abbild 2 in üblicher Weise segmentiert. Das Ergebnis ist eine Sammlung von in dem Abbild 2 enthaltenen Konturen von Bildinhalten. Die Erfindung betrifft die Frage, welche Kontur 6 von eventuell mehreren im Abbild enthaltenen Konturen eine Hand enthält und welcher Teil dieser Kontur 6 eine Hand 4 ist.

In einem zweiten Schritt wird der aussichtsreichste Kandidat ausgewählt. Sollten etwa weitere Körperteile ebenfalls im Aufnahmebereich 1 sichtbar sein, so ist zum Beispiel ein ausgestreckter Arm 3 leicht anhand seiner Proportionen, insbesondere am Verhältnis Länge zu Breite, zu erkennen. Ziel ist ein Verkürzen der Rechenzeit, da bei geeigneter Auswahl - z.B. Wahl der längsten Kontur im Bild - bei der verwendeten Anordnung sicher die Arm-Hand-Kontur 6 entdeckt wird. Falls Unsicherheiten auftreten sollten, könnte hier die Liste der Konturen geordnet nach einem geeigneten Merkmal abgearbeitet werden.

In einem dritten Schritt wird auf die Kontur 6 ein Füllalgorithmus ausgehend von jedem Randpunkt der Kontur 6 nach innen angewendet. Man kann sich das so vorstellen, dass vom Rand ausgehend Farbe in die Kontur 6 hineinläuft. Das Ergebnis dieses Schritts ist ein guter Kandidat für den Mittelpunkt des größten einbeschriebenen Kreises. Ziel des dritten Schritts ist eine Verbesserung der Performance, da ein Abarbeiten aller Punkte der Fläche innerhalb der Kontur 6 und jeweils die Berechnung des größten einbeschriebenen Kreises ungleich länger dauern würde.
In einem vierten Schritt wird nun in einem definierten, relativ zur Gesamtfläche der Kontur 6 kleinen Gebiet für jeden Punkt der größtmögliche einbeschriebene Kreis 7 berechnet. Üblicherweise kommt dieser Kreis an der Verbindung zwischen Daumen und Zeigefinger 8 und der Handwurzel 9 zum Anliegen. Dadurch ist die Hand 4 und ihre Position erfasst.
Insbesondere wird dabei das Handzentrum bestimmt, etwa als Mittelpunkt des Kreises.

In einem fünften Schritt erfolgt schließlich ausgehend vom Handzentrum ein Test auf weitere charakteristische und notwendige Handmerkmale.

Das auf die oben beschriebene Weise gefundene Handzentrum hat sich als sehr gut reproduzierbar erwiesen und ist daher von zentraler Bedeutung für die Qualität der relativ dazu berechneten Merkmale. Ohne ein so definiertes Handzentrum wäre
1. die Hand im Bild viel schwieriger auszumachen und
2. die Qualität der berechneten Merkmale erheblich schlechter.

Vom Kreis 4 ausgehend können zum Beispiel die Handgelenke und die Fingerpositionen bestimmt werden, um daraus die charakteristischen Merkmale der Hand 4 zu berechnen. Insbesondere kann dann eine übliche Handerkennung vorgenommen werden.

Durch den Kreis 7 lässt sich auch die Position des Handgelenks sehr gut bestimmen und damit auch die Punkte zur Trennung der Kontur der Hand 4 von der Kontur des Arms 3.

Allen Ausführungsformen der Erfindung ist der Vorteil zu Eigen, dass vom Benutzer nur ein minimaler Aufwand gefordert wird. Er muss lediglich seine Hand in der typischen Authentisierungsgeste im Aufnahmebereich 1 positionieren. Ein unerwünschter Kontakt zu auch von anderen Benutzern berührten Oberflächen oder das aufwendige Einführen der Hand in eine Kammer entfällt.

## Patentansprüche

1. Anordnung zur Erfassung einer an einem Arm (3) befindlichen Hand (4), der ein zumindest teilweises Abbild (2) der Hand (4) und des Arms (3) zuführbar ist,
**dadurch gekennzeichnet,**
**dass** die Anordnung Mittel zum Auffinden eines Teils des Abbilds (2) mit einem großen Verhältnis von Inhalt zu Außenseiten aufweist.

2. Anordnung nach Anspruch 1,
**dadurch gekennzeichnet,**
**dass** das große Verhältnis von Inhalt zu Außenseiten in etwa das größte Verhältnis von Inhalt zu Außenseiten ist.

3. Anordnung nach Anspruch 1 oder 2,
**dadurch gekennzeichnet,**
**dass** die Mittel zum Auffinden so ausgebildet sind, dass dem Abbild (2) ein geometrisches Objekt (4) einbeschreibbar ist.

4. Anordnung nach zumindest Anspruch 3,
**dadurch gekennzeichnet,**
**dass** das geometrische Objekt (4) dem Abbild (2) mehrfach an unterschiedlichen Positionen einbeschreibbar ist und der Teil des Abbilds (2) dort auffindbar ist, wo das geometrische Objekt (4) am größten einbeschreibbar ist.

5. Anordnung nach zumindest Anspruch 3,
**dadurch gekennzeichnet,**
**dass** das geometrische Objekt (4) ein Kreis, ein Rechteck, ein Polygon, ein Quader und/oder ein Rotationskörper, insbesondere eine Kugel, ist.

6. Anordnung nach zumindest einem der vorhergehenden Ansprüche,
**dadurch gekennzeichnet,**
**dass** die Anordnung Mittel zum Bestimmen der Position der Hand (4) aufgrund der Position des Teils des Abbilds (2) aufweist.

7. Anordnung nach zumindest einem der vorhergehenden Ansprüche,
**dadurch gekennzeichnet,**
**dass** die Anordnung eingerichtet ist, eine Handerkennung durchzuführen.

8. Anordnung nach zumindest einem der vorhergehenden Ansprüche,
**dadurch gekennzeichnet,**
**dass** die Anordnung Mittel zum Identifizieren der Hand (4) in einer Umgebung aufweist.

9. Anordnung nach zumindest einem der vorhergehenden Ansprüche,
**dadurch gekennzeichnet,**
**dass** das Handzentrum der Hand (4) bestimmbar ist.

10. Verfahren zum Erfassen einer an einem Arm (3) befindlichen Hand (4), bei dem zumindest teilweise ein Abbild (2) der Hand (4) und des Arms (3) aufgenommen wird und das insbesondere mit einer Vorrichtung nach einem der Ansprüche 1 bis 9 ausgeführt wird,
**dadurch gekennzeichnet,**
**dass** eines Teil des Abbilds (2) mit einem großen Verhältnis von Inhalt zu Außenseiten aufgefunden wird.

11. Programmprodukt für eine Datenverarbeitungsanlage, das Softwarecodeabschnitte enthält, mit denen ein Verfahren nach Anspruch 10 auf einer Datenverarbeitungsanlage ausgeführt werden kann.
